# EUROPEAN PATENT APPLICATION

(11) **EP 0 617 017 A1**
(43) Date of publication of application: **28.09.1994**
(21) Application number: 94200693.3
(22) Date of filing: 17.03.1994
(51) Int. Cl.: C07D 205/08, C07F 5/02, C07F 7/18

(54) **A process for the preparation of cyclohexene derivatives**

(30) Priority: 20.03.1993 GB 9305805
(71) Applicant: GLAXO S.p.A., I-37100 Verona (IT)
(72) Inventor: Biondi, Stefano, I-37100 Verona (IT); Rossi, Tino, I-37100 Verona (IT); Contini, Stefania Anna, I-37100 Verona (IT)
(74) Representative: Filler, Wendy Anne, Dr.

(57) **Abstract**

1. A process for preparing the compounds of formula (I) 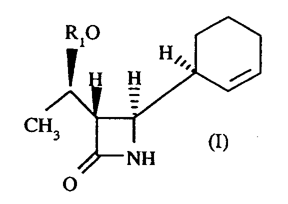
wherein R₁ is a hydroxyl protecting group which comprises reacting the azetidinone (II) 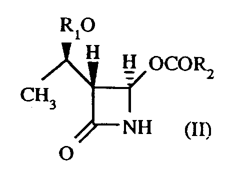
wherein R₁ is a hydroxyl protecting group and R₂ is C₁₋₄alkyl or an optionally substituted phenyl group with the borane derivative (III) 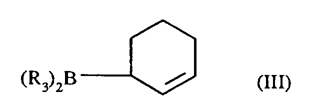
wherein R₃ is isopinocampheyl or 2-methylcyclohexyl in the presence of a Lewis acid and in an aprotic solvent which compounds are useful intermediates for the preparation of antibacterial compounds.

## Description

This invention relates to a process for the preparation of cyclohexene derivatives which are useful in the preparation of compounds having antibacterial activity.

European Patent Application EPA No. 0416953A describes a novel class of tricyclic antibacterial agents and process for their preparation. Further the specification teaches that useful intermediates for preparing preferred antibacterial compounds specifically described and claimed therein are the cyclohexene derivatives of formula (I) 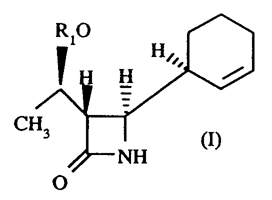
wherein R₁ is a hydroxyl protecting group. The compounds of formula (I) are also described and claimed EPA No. 0466509A2. The present invention relates to a novel and improved process for preparing cyclohexene derivatives of formula (I).

Thus the present invention provides a process for the preparation of compounds of formula (I) which comprises reaction of the azetidinone (II) wherein R₂ is C₁₋₄alkyl or an optionally substituted phenyl group, and R₁ is a hydroxyl protecting group, 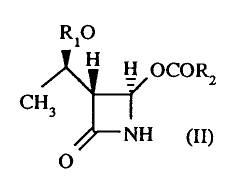
with the borane derivative (III) wherein R₃ is isopinocampheyl or 2-methylcyclohexyl. 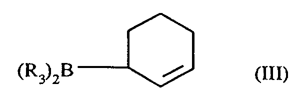
in the presence of a suitable Lewis acid.

Examples of suitable Lewis acids include dialkyl Zinc e.g. diethyl Zinc, trialkyl aluminium e.g. triethylaluminium, isopropoxides such as aluminium triisopropoxide or titanium tetraisopropoxide or alkyl boranes such as triethylborane. The reaction is carried out in an aprotic solvent such as an ether e.g. tetrahydrofuran or a hydrocarbon such as hexane , or halohydrocarbon e.g. chlorobenzene or mixtures thereof and preferably at a temperature within the range -30⁰ to +30⁰.

The borane derivative (III) may be prepared by reaction of the corresponding borane (R₃)₂BH (IV) with cyclohexadiene in a solvent such as a hydrocarbon e.g. hexane or chlorobenzene or an ether e.g. tetrahydrofuran at a temperature within the range - 30 to +30^{o}.

The borane (IV) may be prepared by the reaction of borane dimethylsulphide complex with 1-S-(-)-alpha-pinene, or 1-methylcyclohexene. This reaction is preferably carried out in a solvent such as an ether e.g. tetrahydrofuran or diethyl ether.

The azetidinones of formula (II) are either known compounds or may be prepared by analogous methods to that described for known compounds. Preferred azetidinones for use in the reaction include compounds of formula (II) wherein R₁ is a trialkylsilyl group e.g. triC₁₋₄alkylsilyl group such as trimethyl silyl or t-butyldimethylsilyl and R₂ is a C₁₋₄alkyl e.g. methyl or a phenyl group., more particularly methyl. A particularly useful azetidinone for use in the reaction is that wherein R₁ is t-butyldimethylsilyl and R₂ is methyl.

The process of the present invention has the advantage over known processes for preparing compounds of formula (I) in that it gives the required steroisomer in high yield and in fewer process steps.

In order that the invention may be more fully understood the following example is given by way of illustration only.

### Example 1

### (3S,4R)-4-[(3'S)-Cyclohexene-3-yl]-3-[(1R)-1-t-butyldimethylsilyl oxyethyl]azetidin-2-one

(1)-(S)-α-Pinene(82%ee : 300ml) was added dropwise over 1hr under a slow stream of nitrogen to a solution of borane dimethylsulphide complex (800mmols) in dry tetrahydrofuran (400ml) at 0⁰ and under nitrogen. The mixture was stirred for 4 hr at 0⁰ and then allowed to stand for 2hr at 0⁰. The supernatant liquid was removed via a cannula, anhydrous tetrahydrofuran (200ml) added to the white precipitate and the mixture stirred for 10min. The solid was then allowed to separate out and the liquid phase again removed by cannula. The precipitate was then suspended in dry tetrahydrufuran (800ml) and the stirred mixture cooled to -25⁰. 1,3-Cylcohexadiene (95ml) was then added over 30min and the mixture stirred for a further 12hr at -25⁰. The temperature of the solution was raised to 10⁰ and a solution of (3R,4R)-4-acetoxy-3[(R)-(t-butyldimethylsilyloxy) ethyl]-2-azetidinone (46g) in dry tetrahydrofuran (100ml) was added. A 1M solution of diethyl zinc in hexane (200ml) was added over 20min, the reaction mixture stirred for a further 3.5hr. and then added to a pH3 buffered aqueous solution (200ml) prepared from citric acid and sodium citrate and stirred for 1h. The aqueous phase was extracted with ethyl acetate (1 x 1l; 1 x 500ml). The combined ethyl acetate extracts were washed and with a pH3 aqueous buffered solution (200ml), a saturated sodium bicarbonate solution (2 x 400ml) water (200ml) and brine (2 x 200ml). The solution was dried over anhydrous sodium sulphate and the solvent removed under reduced pressure. The resultant oil was extracted with acetonitrile (1 x 200ml and 4 x 100ml), the combined acetonitrile extracts were concentrated to 2/3 of the original volume and washed with n-hexane (3 x 50ml). The acetonitrile solution was concentrated under presssure and the resultant oil dissolved in methanol (3 volumes) at room temperature. Water (1.3 volumes) was added over 30 min to the vigourously stirred methanol solution to give the title compound as a white solid (22g). The combined n-hexane washings were extracted with acetonitrile (200ml) and the acetonitrile solution concentrated. The resultant oil was dissolved in methanol (3 volumes) and water (1.3 volumes) added over 30 min to the sitrred solution to give another 7g of the title compound.
¹H-NMR (CDCl₃, ppm SiMe4): 6.24 (s, broad), 5.80(m), 5.60(m), 4.13(m), 3.44(dd), 2.84(m), 2.20(m), 1.79(m), 1.52(m), 1.25(m), 1.22(d), 0.87(s), 0.07(s), 0.06(s).
IR (CDCl₃): 3416 (NH str), 1757 (C=O b-lactam, str) 1601 (C=C str).
HPLC: column spherisorb S5-CN, 20X0.46cm; flux 2ml/min; wavelength 220nm; eluant:hexane/acetonitrile/ethanol 99/0.5/0.5
elution time = 8.6min
HPLC: column hypersil BDS C18, 20X0.46cm; flux 1.5ml/min; wavelength 205 nm; elutant methanol/acetonitrile/water 50/30/20.
elution time = 33min.

### Example 2

### (3S,4R)-4-[(3'S)-Cyclohexen-3-yl]-3-[1R)-1-tert-utyldimethylsilyloxyethyl]azetidin -2-one

Borane dimethylsulphide complex (4ml) was dissolved in anhydrous tetrahydrofuran (40ml) at under a nitrogen atmosphere, 1-methylcyclohexene (11 ml) was slowly added and the resulting mixture stirred for 4 hours. The stirring was stopped and the white solid formed allowed to precipitate. The supernatant was removed via a cannula then anhydrous tetrahydrofuran (20ml) was added and the mixture was stirred for 5 minutes. The solid was allowed to precipitate and the supernatant was removed via a cannula. Anhydrous tetrahydrofuran (40ml) and 1,3-cyclohexadiene (10ml) were added and the mixture was stirred for 20 hours. (3S,4R)4-acetoxy-3-[(1R)-(tert-butyldimethylsilyloxy)-ethyl]-azetidin-2-one (2.87g), was added and the solution stirred for 5 minutes, then diethylzinc (1M solution in hexanes, 10ml) was added. After 30 minutes the reaction was quenched with a saturated solution of sodium , potassium tartrate (200ml).The aqueous solution was extracted with diethyl ether (2x200ml). The organic phase was dried over anhydrous sodium sulphate, concentrated to furnish a crude mixture which was chromatographed. Elution with a 1/1 mixture of diethyl ether and cyclohexane gave 3g of product which was shown by nmr studies to consist essentially of the title compound (87% 2.61g).
1H-NMR (400MHz; CDCl3) 5.87 (ma, 1H), 5.81 (m, 1H), 5.61 (m, 1H), 4.15 (m, 1H), 3.46 (dd, 1H), 2.85 (m, 1H), 2.25 (m 1H), 2.00 (m, 2H), 1.85-1.52 (m, 4H), 1.27 (m, 3H), , 0.86 (s, 9H), 0.069 (s, 3H), 0.058 (s, 3H).,
The product of Example 2 may be used directly without further purification to prepare stereospecific antibacterial compounds described in EPA No. 418953A.
The other compounds identified by NMR in the above mentioned reaction product (3g) were:
the corresponding (3S,4S)-[(3'R)-cyclohexen-3-yl]-3-[(1R)-(tert-butyldimethylsilyloxy)-ethyl]-azetidin-2-one (4,6%),
(3S,4R)-3-[(1R)-(tert-butyldimethylsilyloxy)-ethyl]-azetidin-2-one(2%) and 2-methoxycyclohexanol (6.3%).

## Claims

1. A process for preparing compounds of general formula (I) 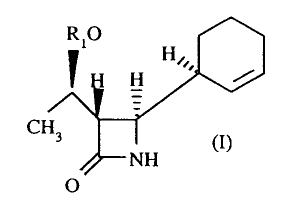 wherein R₁ is a hydroxyl protecting group which comprises reacting the azetidinone (II) 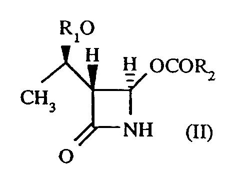 wherein R₁ is a hydroxyl protecting group and R₂ is C₁₋₄alkyl or an optionally substituted phenyl group with the borane derivative (III) 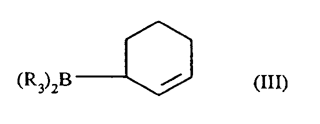 wherein R₃ is isopinocampheyl or 2-methylcyclohexyl in the presence of a Lewis acid and in an aprotic solvent.

2. A process as claimed in Claim 1 wherein the reaction is carried out at a temperature within the range -30⁰ to +30⁰C.

3. A process as claimed in Claims 1 or 2 wherein the Lewis acid selected from a dialkyl zinc, trialkyl aluminium, aluminium isopropoxide, titantium tetraisopropoxide or alkyl borane.

4. A process as claimed in any of Claims 1 to 3 wherein the Lewis acid is diethyl zinc.

5. A process as claimed in any of Claims 1 to 4 wherein R₂ is a methyl group.

6. A process as claimed in any of Claims 1 to 5 wherein R₃ is isopinocampheyl.

7. Compounds of general formula (I) whenever prepared by a process as claimed in any of Claims 1 to 6.

8. Compounds as claimed in Claim 7 wherein R₁ is a trialkylsilyl protecting group.

9. Compound as claimed in Claim 8 wherein R₁ is a t-butyldimethylsilyl group.
